# EUROPÄISCHE PATENTANMELDUNG

(11) **EP 1 382 389 A1**
(43) Veröffentlichungstag der Anmeldung: **21.01.2004**
(21) Anmeldenummer: 03019556.4
(22) Anmeldetag: 30.01.2001
(51) Int. Cl.: B01J 25/00, B01J 37/14, C07C 51/00

(54) **Raney-Kupferkatalysator zur Dehydrierung von Alkoholen**

(30) Priorität: 18.02.2000 EP 00103546
(62) Teilanmeldung aus: 01102003.9
(71) Anmelder: Degussa AG, 40474 Düsseldorf (DE)
(72) Erfinder: Ostgard, Daniel, Dr., 63801 Kleinostheim (DE); Sauer, Jörg, Dr., 48249 Dülmen (DE); Freund, Andreas, Dr., 63571 Gelnhausen (DE); Berweiler, Monika, 63477 Maintal (DE); Höpp, Mathias, Dr., 63584 Gründau (DE); Vanheertum, Rudolf, Dr., 2930 Braschaat (BE); Girke, Walter, 63457 Hanau-Grossauheim (DE)
(74) Vertreter: Weber, Wolfgang, Dr

(57) **Zusammenfassung**

Raney-Kupfer-Katalysator, bei dem die ursprüngliche Legierung in Luftatmosphäre bei Temperaturen höher als 500°C vor der Aktivierung calziniert wurde, wird als Katalysator bei der Dehydrierung von Alkoholen eingesetzt.

## Beschreibung

Die Erfindung betrifft Raney-Kupfer, ein Verfahren zu seiner Herstellung sowie ein Verfahren zur Dehydrierung von Alkoholen.

Es ist bekannt, Diethanolamin zu Iminodiessigsäure zu dehydrieren. (US 5,689,000; WO 96/01146; WO 92/06949; JP-OS 091 55 195; US 5,292,936; US 5,367,112; CA 212 10 20)

Gegenstand der Erfindung ist Raney-Kupfer, welches dadurch gekennzeichnet ist, daß es mit mindestens einem Metall aus der Gruppe Eisen und/oder Edelmetall dotiert ist.

Die Dotierung kann sowohl durch Zulegierung des Dotierungselements zur Raneylegierung, bestehend aus Kupfer und Aluminium, als auch durch Imprägnierung des fertig präparierten Raney-Kupfers mit dem Dotierungselement bewehrstelligt werden.

Das erfindungsgemäße Raney-Kupfer kann die Dotierungselemente in einer Menge von 10 ppm bis 5 Gew.-% enthalten. Die Edelmetalldotierung kann 10 bis 50.000 ppm, vorzugsweise 500 bis 50.000 ppm, betragen. Die Dotierungsmetalle können aus der Gruppe Eisen sowie Palladium, Platin, Gold, Rhenium, Silber, Iridium, Ruthenium und/oder Rhodium ausgewählt werden.

Das erfindungsgemäße Raney-Kupfer kann Meso- und MakroPoren jedoch keine Mikroporen aufweisen.

Die ursprünglich gebildete Legierung kann mehr als 50 % Kupfer enthalten, sodaß der endgültige Katalysator mehr restliches Aluminium enthält als normalerweise unter denselben Aktivierungsbedingungen gefunden wird.

Die ursprünglich gebildete Legierung kann einer Hitzebehandlung in Luftatmosphäre bei Temperaturen oberhalb von 500 °C vor der Aktivierung unterzogen werden.

Die ursprünglich gebildete Legierung kann mehr als 50 % Kupfer enthalten und in Luftatmosphäre einer Hitzebehandlung bei Temperaturen oberhalb 500 °C vor der Aktivierung unterzogen werden.

Die mittlere Teilchengröße des erfindungsgemäßen Raney-Kupfers kann 35 ± 30 µm betragen.

Die mittlere Teichengröße des erfindungsgemäßen Raneykupfers ist von Bedeutung bei der Anwendung bei Oxidationsreaktionen beziehungsweise Dehydrierungsreaktionen von Alkoholen.

Das bekannte Raney-Kupfer bildet bei der mehrfachen Verwendung Granulate (Agglomerate), wodurch das Raney-Kupfer desaktiviert wird.

Das erfindungsgemäße, mit Eisen und/oder Edelmetall dotierte Raney-Kupfer wird nicht durch eine unerwünschte Granulation desaktiviert. Das erfindungsgemäße Raney-Kupfer läßt sich vorteilhafterweise gut filtrieren.

Das erfindungsgemäße Raney-Kupfer zeigt eine höhere Aktivität als das Cr/Raney-Kupfer gemäß EP 0 620 209 A1 oder US 5,292,936 bei der Dehydrierung von Ethylenglykol.

Das erfindungsgemäße Raney-Kupfer enthält weiterhin vorteilhafterweise keine toxischen Metalle, wie zum Beipiel Chrom.

Ein weiterer Gegenstand der Erfindung ist ein Verfahren zur Herstellung des Raney-Kupfers, welches dadurch gekennzeichnet ist, daß man eine Kupfer-Aluminium-Legierung mittels wäßriger Natronlaugenlösung aktiviert, den Katalysator wäscht, in Wasser suspendiert, zu dieser Suspension eine Eisensalz- oder Edelmetallsalz-Lösung hinzugibt, den pH-Wert der Lösung auf einen Wert von 4 bis 11 einstellt, den Katalysator von der Lösung abtrennt und wäscht.

Ein weiterer Gegenstand der Erfindung ist ein Verfahren zur Herstellung des Raney-Kupfers, welches dadurch gekennzeichnet ist, daß man das Dotierungsmetall zusammen mit Kupfer und Aluminium legiert, anschließend mittels wäßriger Natronlaugenlösung aktiviert und den Katalysator wäscht.

Ein weiterer Gegenstand der Erfindung ist ein Verfahren zur katalytische Dehydrierung von Alkoholen zu den entsprechenden Carbonyl-Verbindungen und Carbonsäuren, welches dadurch gekennzeichnet ist, daß man als Katalysator ein mit Eisen oder Edelmetall dotiertes Raney-Kupfer verwendet.

Das erfindungsgemäße Verfahren zur Dehydrierung von Alkoholen kann zur Dehydrierung von Glykolen und/oder Aminoalkoholen verwendet werden. Dabei kann der Katalysator in Form einer Suspension eingesetzt werden.

Die Alkohole, die erfindungsgemäß dehydriert werden können, können ein- oder mehrwertige Alkohole sein. Sie können inklusive von Polyetherglykolen aliphatische, cyclische oder aromatische Verbindungen sein, die mit einer starken Base zu dem Carboxylat reagieren.

Hierbei ist es notwendig, daß der Alkohol und das resultierende Carboxylat in stark basischer Lösung stabil sind, und der Alkohol wenigstens etwas in Wasser löslich ist.

### Geeignete primäre, einwertige Alkohole können umfassen:

Aliphatische Alkohole, die verzweigte, gradkettige, cyclische oder aromatische Alkohole, wie zum Beispiel Benzylalkohol, sein können, sein, wobei diese Alkohole mit verschiedenen, basenstabilen Gruppen substituiert sein können.

Geeignete aliphatische Alkohole können Ethanol, Propanol, Butanol, Pentanol oder ähnlich sein.

Erfindungsgemäß können Glykole zu Carbonsäuren oxidiert beziehungsweise dehydriert werden. Glykole können zum Beispiel sein:
Ethylenglykol
Propopylenglykol
1,3-Propandiol
Butylenglykol
Butandiol -1,4

So kann man zum Beispiel Ethylenglykol zu Glykolsäure (Monocarbonsäure) dehydrieren und durch anschliessende Umsetzung mit KOH die Dicarbonsäure Oxalsäure herstellen.

Aminoalkohole können ebenfalls mit dem erfindungsgemäß, dotierten Raney-Kupfer zu den entsprechenden Aminocarbonsäuren dehydriert werden. Die Aminoalkohole können 1 bis 50 C-Atome aufweisen.

So kann man beispielsweise
N-Methylethanolamin zu Sarkosin; THEEDA (Tetrahydroxyethylenthylendiamin) zum Tetranatriumsals von EDTA (Ethylendiamtetraacetat);
Monoethanolamin zu Glycin;
Diethanolamin zu Iminodiessigsäure;
3-Amino-1-propanol zu Beta-Alanin;
2-Amino-1-butanol zu 2-Aminobuttersäure dehydrieren.

In einer Ausführungsform der Erfindung können mit dem erfindungsgemäßen Verfahren Aminoalkohole der Formel 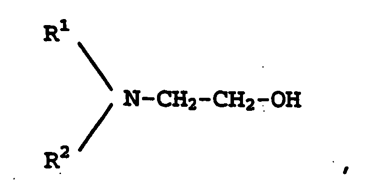 in der R¹ und R² jeweils Wasserstoff; Hydroxyethyl; -CH₂CO₂H; eine Alkylgruppe mit 1 bis 18 C-Atomen; eine Aminoalkylgruppe mit 1 bis 3 C-Atomen; eine Hydroxyalkylaminoalkyl-Gruppe mit 2 bis 3 C-Atomen sowie Phosphonomethyl bedeuten, dehydriert werden.

Die Aminoalkohole, die erfindungsgemäß eingesetzt werden können, sind bekannt. Wenn R¹ und R² gleich Wasserstoff sind, dann ist der Aminoalkohol Diethanolamin.

Wenn R¹ und R² Hydroxyethyl sind, dann ist der Aminoalkohol Triethanolamin. Die resultierenden Aminocarbonsäure salze dieser Ausgangsaminoalkohole sollten die Salze von Glycin, Iminodiessigsäure, beziehungsweise Nitrilotriessigsäure sein. Weitere Aminoalkohole umfassen N-methylethanolamin, N,N-dimethylethanolamin, N-ethylethanolamin, N-isopropylethanolamin, N-butylethanolamin, N-nonylethanolamine, N-(2-aminoethyl) ethanolamin, N-(3-aminopropyl)ethanolamin, N,N-diethylethanolamin, N,N-dibutylethanolamin, N-methyldiethanolamin, N-ethyldiethanolamin, N-isopropyldiethanolamin, N-butyldiethanolamin, N-ethyl,N-(2-aminoethyl)-ethanolamin, N-methyl , N-(3-aminopropyl) ethanolamin, tetra (2-hydroxyethyl) ethylenediamin und ähnliche.

Weitere Beispiele für Aminocarbonsäuresalze sind die Salze von N-methylglycin, N,N-dimethylglycin,N-ethylglycin, N-isopropylglycin, N-butylglycin, N-nonylglycin, N-(2-aminoethyl)glycin, N-3-aminopropyl)glycin, N,N-diethylglycin, N,N-dibutylglycin, N-methyliminodiessigsäure, N-ethyliminodiessigsäure, N-isopropyliminodiessigsäure, N-butyliminodiessigsäure, N-ethyl, N-(2-aminoethyl)glycin, N-methyl-N-(3-aminopropyl) glycin, Ethylenediaminetetraessigsäure, und so weiter.

R¹ oder R² können ebenfalls eine Phosphonomethyl-Gruppe sein, wobei die Ausgangsaminoverbindung N-phosphonomethylethanolamin und die resulierend Aminosäure N-phosphonomethylglycin sein können. Wenn von R¹ oder R² ein R = Phosphonomethyl und das andere R = -CH₂CH₂OH ist, wäre die resultierende Aminosäure N-phosphonomethyliminodiessigsäure, die zu N-phosphonomethylglycin auf bekanntem Wege umgewandelt werden kann. Wenn von R¹ oder R² ein R = Phosphonomethyl ist und das andere R = eine Alkylgruppe ist, wäre die resultierende Säure N-alkyl-N-phosphonomethylglycin, das zu N-phosphonomethylglycin entsprechend dem U.S. Patent 5,068,404 weiter umgewandelt werden kann.

Das erfindungsgemäße Verfahren kann bei einer Temperatur von 50 bis 250 °C, bevorzugt 80 bis 200 °C, und bei einem Druck von 0,1 bis 200 bar, bevorzugt Normaldruck bis 50 bar, durchgeführt werden.

Der Druck ist notwendig, weil die Alkohole einen hohen Dampfdruck aufweisen. Bei dem Ablassen des Wassestoffes würde bei einem zu niedrigen Druck auch der Alkohol abgelassen werden.

### Beispiel 1: (Herstellung des erfindungsgemäßen Katalysators)

Eine Legierung, bestehend aus 50%Cu/50%A1 wird mit einer wässrigen Natronlaugelösung aktiviert. Der entsprechende Katalysator wird gewaschen bis das Natriumaluminat vollständig entfernt ist. Anschließend wird Hexachloroplatin zu der Suspension des gewaschenen Katalysators gegeben. Der pH-Wert wird eingestellt und die Suspension weiter gerührt. Der dotierte Katalysator wird anschliessend gewaschen. Der Platingehalt des Katalysators ist 1%. Die Aktivität dieses Katalysator für Ethyleneglycoldehydrierung ist 299 ml Wasserstoff pro Stunde pro Gram Katalysator (siehe Beispiel 3).

### Beispiel 2: (Herstellung der erfindungsgemäßen Katalysators)

Eine Legierung, bestehend aus 50%Cu/50%A1, wird mit einer wässrigen Natronlaugelösung aktiviert. Der entsprechende Katalysator wird gewaschen bis das Natriumaluminat vollständig entfernt ist. Anschließend wird Eisen III Chlorid zu der Suspension des gewaschenen Katalysators gegeben. Der pH-Wert wird eingestellt und die Suspension weiter gerührt. Der dotierte Katalysator wird anschliessend gewaschen. Der Eisengehalt des Katalysators ist 3%.

### Beispiel 3

Die Dehydrierung von Ethylenglycol zu Natriumglycolat und Natriumoxalat mittels dem aktivierten Kupferkatalysator gemäß Beispiel wird bei 108 °C und atmosphärischem Druck durchgeführt. Zuerst wird 70 ml Ethylenglycol zu einer heterogenen Suspension von 8 Gramm Katalysator und 70 ml einer wässerigen Natronlaugelösung gegeben. Die Suspension wird bei 400 upm gerührt. Die Reaktionsgeschwindigkeit wird an Hand der entwickelten Menge Wasserstoff zwischen 30 und 90 Minuten seit Beginn der Reaktion gemessen. Die Ergebnisse werden als ml Wasserstoff pro Stunde pro Gramm Katalysator angegeben. Die Aktivität dieses Katalysators für die Dehydrierung von Ethylenglykol ist 299 ml Wasserstoff pro Stunde pro Gramm Katalysator.

### Beispiel 4 (Vergleichsbeispiel)

Eine Legierung, bestehend aus 50%Cu/50%A1, wird mit einer wässrigen Natronlaugelösung aktiviert. Der entsprechende Katalysator wird gewaschen, bis das Natriumaluminat vollständig entfernt ist. Die Aktivität dieses Katalysators ist bei der Ethylenglycoldehydrierung 205 ml Wasserstoff pro Stunde pro Gramm Katalysator.

### Beispiel 5 (Vergleichsbeispiel)

Eine 50&Cu/50%A1 Legierung wird mit einer wässerigen Natronlaugelösung aktiviert. Der entsprechende Katalysator wird gewaschen, bis das Natriumaluminat vollständig entfernt ist. Chromnitrat wird zu der Suspension des gewaschenen Katalysators gegeben, der pH-Wert wird reguliert, die Suspension weiter gerührt und der dotierte Katalysator noch einmal gewaschen. Der Gehalt an Chrom in dem Katalysator ist 2000 ppm. Die Aktivität des Katalysators bei der Ethylenglycoldehydrierung ist 253 ml Wasserstoff pro Stunden pro Gram Katalysator.

### Beispiel 6 (Vergleichsbeispiel)

Eine Cu/Al/V Legierung wird mit einer wässerigen Natronlaugenlösung aktiviert. Der entsprechende Katalysator wird gewaschen, bis das Natriumaluminat vollständig entfernt ist. Der Gehalt an V in dem Katalysator beträgt 1%. Die Aktivität des Katalysators bei der Ethylenglycoldehydrierung ist 253 ml Wasserstoff pro Stunde pro Gram Katalysator.

### Beispiel 7

Herstellung von Iminodiessigsäure mit Platin auf Raney-Kupfer als Katalysator.

Das Beispiel illustriert die Umsetzung von Diethanolamin (DEA) zum Natrium-Salz der Iminodiessigsäure (IDA) mit Pt-dotiertem Raney-Kupfer als Katalysator.

Die Versuche werden in einem 2 1 Autoklav (Fa. Büchi) durchgeführt. Der Autoklav ist mit einem Begasungsrührer ausgestattet, der standardmäßig bei 500 min-1 betrieben wird. Der Autoklav ist mit einem Doppelmantel ausgestattet. Die Temperatur im Autoklaven kann über einen Ö1-Thermostaten eingestellt werden.

In dem Autoklaven wird folgender Ansatz vorgelegt:

| | |
|---|---|
| 318.8 g | Diethanolamin (3 mol) |
| | |
| 508 g | wässrige NaOH-Lösung (50 Gew.-%, 6.3 mol NaOH) |
| | |
| 64 g | erfindungsgemäßer Katalysator: 1% Pt auf Raney-Kupfer, unter Wasser gelagert |
| | |
| 370 g | H₂O, ultraschall-entgast |

Der Autoklav wird mit Stickstoff auf 10 bar aufgedrückt und auf Reaktionstemperatur gebracht (TR=160°C). Nach Anspringen der Reaktion wird der entstehende Wasserstoff abgelassen, wobei die freigesetzte Menge über eine Trocken-Gasuhr bestimmt wird. Die Reaktion wird nach einer Dauer von 5 h abgebrochen und der Autoklav abgekühlt. Die Reaktionsprodukte werden mit entgastem Wasser aus dem Autoklaven gespült, der Katalysator abfiltriert und die Produkte der Dehydrierung durch ionenchromatographische Auftrennung analysiert.

Der eingesetzte Katalysator kann - wie in der Tabelle 1 dargestellt ist - ohne nennenswerten Aktivitätsverlust mehrfach rezykliert werden.

**Tabelle 1**

| Umsetzung von Diethanolamin an Pt-dotiertem Raney-Kupfer | |
|---|---|
| **Anzahl der Ansätze mit Katalysator** | **Ausbeut» IDA [mol %]** |
| 1 | 94.3 |
| 2 | 92.5 |
| 3 | 98.6 |
| 4 | 96.8 |
| 5 | 95.0 |
| 6 | 94.7 |
| 7 | 90.9 |
| 8 | 91.8 |
| 9 | 93.4 |
| 10 | 95.8 |
| 11 | 97.7 |
| 12 | 93.5 |
| 13 | 95.7 |
| 14 | 92.6 |
| 15 | 90.0 |
| 16 | n.b |
| 17 | n.b. |
| 18 | 95.2 |

### Beispiel 6

Herstellung von Iminodiessigsäure mit Eisen auf Raney-Kupfer als Katalysator.

In einem 2 l Autoklaven wird folgender Ansatz vorgelegt:

| | |
|---|---|
| 318.8 g | Diethanolamin (3 mol) |
| | |
| 508 g | wässrige NaOH-Lösung (50 Gew.-%, 6.3 mol NaOH) |
| | |
| 64 g | erfindungsgemäßer Katalysator: 3% Fe auf Raney-Kupfer, unter Wasser gelagert |
| | |
| 370 g | H₂O, ultraschall-entgast |

Der Versuch wird analog zu Beispiel 5 durchgeführt. Dabei werden die in Tabelle 2 aufgelisteten Ausbeuten erzielt, Eine Desaktivierung des Katalysators ist auch nach mehrfachem Einsatz des Katalysators nicht zu beobachten.

**Tabelle 2**

| Umsetzung von Diethanolamin an Fe-dotiertem Raney-Kupfer | |
|---|---|
| **Anzahl der Ansätze mit Katalysator** | **Ausbeute IDA [mol %]** |
| 1 95.3 | |
| 2 | 99.1 |
| 3 | 99.0 |
| 4 | n.b. |
| 5 | n.b. |
| 6 | 91.9 |
| 7 | n.b. |
| 8 | n.b. |
| 9 | n.b. |
| 10 | 93.7 |
| 11 | n.b. |
| 12 | n.b. |
| 13 | n.b. |
| 14 | 94.0 |

### Beispiel 7

### Vergleichsbeispiel

Herstellung von Iminodiessigsäure an undotiertem Raney-Kupfer.

Reines Raney-Kupfer (Degussa-Katalysator BFX 3113W) wird unter den Bedingungen von Beispiel 5 eingesetzt. Das Raney-Kupfer zeigt schon nach wenigen Ansätzen eine deutliche Deaktivierung. (Tabelle 3)

**Tabelle 3**

| Umsetzung von Diethanolamin an Raney-Kupfer | |
|---|---|
| **Anzahl der Ansätze mit Katalysator** | **Ausbeute IDA [mol %]** |
| 1 | 91.6 |
| 2 | 82.8 |
| 3 | 68.3 |
| 4 | 51.3 |

### Beispiel 8

Herstellung von Glycin mit Platin auf Raney-Kupfer als Katalysator.

In dem 2 l Autoklaven wird folgender Ansatz vorgelegt:

| | |
|---|---|
| 307 g | Monoethanolamin (5 mol) |
| | |
| 420 g | wässrige NaOH-Lösung (50 Gew.-%, 5.25 mol NaOH) |
| | |
| 64 g | erfindungsgemäßer Katalysator: 1% Pt auf Raney-Kupfer, unter Wasser gelagert |
| | |
| 400 g | H₂O; ultraschall-entgast |

Der Versuch wird analog zu Beispiel 5 durchgeführt. Dabei werden die in Tabelle 4 aufgelisteten Ausbeuten erzielt. Eine Desaktivierung des Katalysators ist auch nach mehrfachem Einsatz des Katalysators nicht zu beobachten.

**Tabelle 4**

| Umsetzung von Monoethanolamin an Pt-dotiertem Raney-Kupfer | |
|---|---|
| **Anzahl der Ansätze mit Katalysator** | **Ausbeute Glycin [mol %]** |
| 1 | 98.5 |
| 2 | 97.5 |
| 3 | n.b |
| 4 | n.b. |
| 5 | 98.1 |

### Beispiel 9

Herstellung von β-Alanin mit Platin auf Raney-Kupfer als Katalysator.

In dem 2 l Autoklaven wird folgender Ansatz vorgelegt:

| | |
|---|---|
| 380 g | 3-Amino-1-propanol (5 mol) |
| | |
| 422 g | wässrige NaOH-Lösung (50 Gew.-%, 5.25 mol NaOH) |
| | |
| 64 g | erfindungsgemäßer Katalysator: 1% Pt auf Raney-Kupfer, unter Wasser gelagert |
| | |
| 250 g | H₂O; ultraschall-entgast |

Der Versuch wird analog zu Beispiel 5 durchgeführt. Dabei werden die in Tabelle 5 aufgelisteten Ausbeuten erzielt. Eine Desaktivierung des Katalysators ist auch nach mehrfachem Einsatz des Katalysators nicht zu beobachten.

**Tabelle 5**

| Umsetzung von 3-Amino-1-propanol an Pt-dotiertem Raney-Kupfer | |
|---|---|
| **Anzahl der Ansätze mit Katalysator** | **Ausbeute β-Alanin [mol %]** |
| 1 | 98.2 |
| 2 | 98.5 |
| 3 | n.b. |
| 4 | n.b. |
| 5 | 98.3 |

### Beispiel 10

Herstellung von 2-Amino-Buttersäure mit Platin auf Raney-Kupfer als Katalysator.

In dem 2 l Autoklaven wird folgender Ansatz vorgelegt:

| | |
|---|---|
| 460 g | 2-Amino-1-butanol (5 mol) |
| | |
| 392 g | wässrige NaOH-Lösung (50 Gew.-%, 5.25 mol NaOH) |
| | |
| 64 g | erfindüngsgemäßer Katalysator: 1% Pt auf Raney-Kupfer, unter Wässer gelagert |
| | |
| 140 g | H₂O; ultraschall-entgast |

Der Versuch wird analog zu Beispiel 5 durchgeführt. Dabei werden die in Tabelle 6 aufgelisteten Ausbeuten erzielt. Eine Desaktivierung des Katalysators ist auch nach mehrfachem Einsatz des Katalysators nicht zu beobachten.

**Tabelle 6**

| Umsetzung von. 2-Amino-1-butanol, an Pt-dotiertem Raney-Kupfer | |
|---|---|
| Anzahl der Ansätze mit Katalysator | Ausbeute 2-Amino-1-Buttersäure [mol %] |
| 1 | 99.2 |
| 2 | 98.1 |
| 3 | n.b. |
| 4 | n.b. |
| 5 | 98.9 |

Figur 1 zeigt den Vorteil des erfindungsgemaßen Katalysators am Beispiel der Dehydrierung beziehungsweise der Umsetzung von Diethanolamin zu Iminodiessigsäure.

Der erfindungsgemäße Katalysator zeigt eine deutlich höhere Standzeit als der undotierte Raney-Katalysator.

## Patentansprüche

1. Ein Raney-Kupfer-Katalysator, bei dem die ursprüngliche Legierung in Luftatmosphäre bei Temperaturen höher als 500 °C vor der Aktivierung calziniert wurde.

2. Verfahren zur katalytischen Dehydrierung von Alkoholen zu ihren entsprechenden Carbonyl-Verbindungen und Carbonsäuren, wobei ein Raney-Kupfer-Katalysator gemäß Anspruch 1 eingesetzt wird.
